Europäisches Patentamt

European Patent Office

Office européen des brevets

(19)

(11) Veröffentlichungsnummer : **0 502 921 B1**

(12)                 **EUROPÄISCHE PATENTSCHRIFT**

(45) Veröffentlichungstag der Patentschrift :
**29.09.93 Patentblatt 93/39**

(51) Int. Cl.$^5$ : **C08F 4/54**

(21) Anmeldenummer : **91900028.1**

(22) Anmeldetag : **19.11.90**

(86) Internationale Anmeldenummer :
**PCT/EP90/01977**

(87) Internationale Veröffentlichungsnummer :
**WO 91/08238 13.06.91 Gazette 91/13**

(54) **NEUE STARTERSYSTEME FÜR DIE POLYMERISATIONSAUSLÖSUNG ETHYLENISCH UNGESÄTTIGTER VERBINDUNGEN UND IHRE VERWENDUNG.**

(30) Priorität : **27.11.89 DE 3939164**

(43) Veröffentlichungstag der Anmeldung :
**16.09.92 Patentblatt 92/38**

(45) Bekanntmachung des Hinweises auf die
Patenterteilung :
**29.09.93 Patentblatt 93/39**

(84) Benannte Vertragsstaaten :
**AT BE CH DE DK ES FR GB GR IT LI LU NL SE**

(56) Entgegenhaltungen :
**EP-A- 0 085 944**

(73) Patentinhaber : **Henkel
Kommanditgesellschaft auf Aktien
D-40191 Düsseldorf (DE)**

(72) Erfinder : **RITTER, Wolfgang
Am Bandenfeld 74
D-5657 Haan (DE)**

(74) Vertreter : **von Kreisler, Alek, Dipl.-Chem. et al
Patentanwälte Von Kreisler-Selting-Werner,
Postfach 10 22 41, Bahnhofsvorplatz 1
D-50462 Köln (DE)**

EP 0 502 921 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

EP 0 502 921 B1

## Beschreibung

Die Erfindung betrifft Startersysteme für die Polymerisationsauslösung ethylenisch ungesättigter Verbindungen auf Basis von Sauerstoff-reaktiven Organo-Borverbindungen, die in Kombination mit einem Trägermaterial vorliegen. Die neuen Startersysteme eignen sich prinzipiell für beliebige Anwendungsgebiete - beispielsweise also für den technischen oder handwerklichen Arbeitsbereich - sie haben jedoch eine besondere Bedeutung für die Verwendung auf dem Gebiet der reaktiven Klebstoff- bzw. Zement-Systeme des medizinischen und/oder zahnmedizinischen Anwendungsbereiches, im zuletzt genannten Gebiet insbesondere auch für Füllmassen.

Es besteht eine umfangreiche Literatur zur Polymerisationsauslösung in ethylenische Doppelbindungen enthaltenden ReaktionsSystemen durch Organo-Borverbindungen, die durch Einwirkung von Sauerstoff, insbesondere durch Lufteinwirkung zum reaktiven Starter aktiviert werden. Entsprechende Vorschläge des Standes der Technik betreffen sowohl das Gebiet technischer Klebstoffsysteme als auch solcher Komponenten, die für den medizinischen, insbesondere chirurgischen und/oder zahnmedizinischen Einsatz gedacht sind. Eine Mehrzahl vorveröffentlichter Vorschläge der Anmelderin schildern die hier gegebenen Probleme und die umfangreichen wissenschaftlichen und praktischen Arbeiten, die insbesondere auch von dritter Seite hier bisher geleistet worden sind. Verwiesen wird hierzu auf die nachfolgenden, auf die Anmelderin zurückgehenden Druckschriften und das darin zitierte druckschriftliche Material von dritter Seite - Bezug genommen wird dabei im nachfolgenden auf die jeweiligen deutschen Offenlegungsschriften (DE-A1) der Anmelderin, soweit nicht ausdrücklich anders angegeben:

30 41 843, 30 41 904 (C2), 31 43 945, 32 01 780, 32 04 504 und insbesondere die 32 29 635.

Insbesondere die zuletzt genannte Druckschrift der Anmelderin beschäftigt sich mit Bindemittelsystemen, die auf Basis körperverträglicher, insbesondere körperresorbierbarer Komponenten aufgebaut sind und damit Kleber- bzw. Zementfunktion im chirurgischen und/oder zahnmedizinischen Bereich ebenso übernehmen können wie sie zur Herstellung von gewünschtenfalls auch körperresorbierbaren Formteilen für eine Implantation in den menschlichen oder tierischen Körper geeignet sind. Bei der Kleberfunktion kommt sowohl das Kleben von körpereigenem Gewebe, insbesondere Hartgewebe in Betracht wie die Verklebung solchen Gewebes zusammen mit Kunststoff und/oder Metall.

Die Erfindung geht demgegenüber von der folgenden Aufgabe aus: Es sollte ein Startersystem für die Polymerisationsauslösung auf Basis von Sauerstoff-reaktiven Organo-Borverbindungen gefunden werden, das sich durch eine Optimierung in Herstellung, Lagerfähigkeit und Gebrauchsdauer und im Verhalten im praktischen Einsatz auszeichnet. Die Erfindung will insbesondere Trägersysteme für die Abmischung mit den durch Luftzutritt aktivierbaren Organo-Borverbindungen zur Verfügung stellen, die auch in Abwesenheit von Lösungs- bzw. Verdünnungsmitteln hinreichend niedrig-viskos sind, die leichte und gleichmäßige Einarbeitung in das zu polymerisierende Gut zu gewährleisten. Gleichzeitig soll der Träger die radikalbildenden Organo-Borverbindungen auch bei Luftzutritt so weitgehend stabilisieren, daß ein längeres Verweilen unter Luftzutritt von beispielsweise mehreren Stunden oder gar auch mehreren Tagen möglich ist und zu keiner substantiellen Aktivitätseinbuße führt. Schließlich - und hier liegt für die praktische Anwendung ein besonders wichtiger Gesichtspunkt - will die Erfindung mit den neuen Startersystemen Polymerisationsauslöser zur Verfügung stellen, die mit den üblichen ethylenisch ungesättigten Klebstoffkomponenten, beispielsweise auf Acrylat- oder Methacrylatbasis zu so hinreichenden Topfzeiten führen, daß der für das praktische Arbeiten benötigte Zeitraum von wenigstens einigen Minuten zuverlässig zur Verfügung steht.

Die technische Lösung dieser erfindungsgemäßen Aufgabe geht von der Feststellung aus, daß bestimmt ausgewählte niedrigviskose Trägermaterialien auf Basis von Oligoestern geeignet sind das gestellte Anforderungsprofil optimal abzudecken. Die im nachfolgenden geschilderten Trägermaterialien auf Oligoester-Basis zeichnen sich noch dazu durch physiologische Unbedenklichkeit aus. Sie werden im lebenden Körper abgebaut und können damit unbedenklich im Rahmen von Klebstoffsystemen, Zementen oder sonstigen Kunststoffmaterialien Verwendung finden, die im medizinischen und zahnmedizinischen Bereich eingesetzt werden.

Gegenstand der Erfindung ist dementsprechend in einer ersten Ausführungsform ein Startersystem für die Polymerisationsauslösung ethylenisch ungesättigter Verbindungen auf Basis von Sauerstoff-reaktiven Organo-Borverbindungen in Kombination mit Sauerstoff-resistenten Trägern, wobei das Kennzeichen der Erfindung darin liegt, daß als Träger in diesem Startersystem Oligoester von niederen Hydroxycarbonsäuren mit 2 bis 10 C-Atomen vorliegen.

Bevorzugte Ausführungsformen werden in den abhängigen Ansprüchen genannt.

Wesentlicher esterbildender Bestandteil dieser Trägermaterialien sind damit Hydroxycarbonsäuren des C-Zahlbereichs von 2 bis 10. Besonders geeignet sind Hydroxycarbonsäuren mit 2 bis 6 C-Atomen, die in Form ausgewählter bestimmter Vertreter dieser Stoffklasse oder auch in Abmischung mehrerer Komponenten aus dieser Gruppe zur Oligoesterbildung eingesetzt sein können. Besonders wichtige Hydroxycarbonsäuren zur

EP 0 502 921 B1

Ausbildung dieses Trägers sind die Glykolsäure und/oder insbesondere die Milchsäure, die in Form ausgewählter Isomeren oder auch als Isomerengemisch verwendet werden kann. Geeignet sind weiterhin die gegebenenfalls isomeren Alpha- oder Beta-Hydroxypropionsäuren, die gegebenenfalls isomeren Alpha-, Beta- oder Gamma-Hydroxybuttersäuren, o-Hydroxybenzoesäure (Salicylsäure), m-Hydroxybenzoesäure und/oder p-Hydroxybenzoesäure (Anissäure). Es können dabei sowohl bestimmte Isomere der genannten Säuren als auch beliebige Gemische zum Einsatz kommen.

Die Oligoester der genannten Hydroxycarbonsäuren können in einer bevorzugten Ausführungsform unter Mitverwendung von monofunktionellen und/oder mehrfunktionellen Reaktanten hergestellt worden sein, die dazu führen, daß als Träger mit Hydroxylgruppen oder mit Carboxylgruppen terminierte Oligoester der niederen Hydroxycarbonsäuren vorliegen. Durch diese mitverwendeten Reaktanten wird einerseits die Regelung des mittleren Molekulargewichtes im Esteroligomeren und damit die Einstellung der angestrebten Viskositätsbereiche ermöglicht. Zum anderen ist durch Auswahl der funktionellen Gruppen der mitverwendeten Koreaktanten die Möglichkeit gegeben, an den Esteroligomeren einheitlich endständige Hydroxylgruppen oder endständige Carboxylgruppen auszubilden, was an sich bei einem Oligomeren einer Hydroxycarbonsäure zunächst ja nicht der Fall ist. Durch Mitverwendung ausgewählter monofunktioneller Koreaktanten können gewünschtenfalls reaktive Endgruppen der Oligoester beseitigt werden, auch die zusätzliche Regelung des Oligomerisationsgrades ist auf diese Weise möglich.

Geeignete monofunktionelle Koreaktanten sind insbesondere Monoalkohole und/oder Monocarbonsäuren, wobei entsprechende Verbindungen mit beschränkter C-Zahl beispielsweise mit bis zu 12 C-Atomen, vorzugsweise mit bis zu 6 C-Atomen bevorzugt sein können. Als Trägermaterial besonders geeignet sind allerdings Oligoester der geschilderten Art, die unter Mitverwendung von mehrwertigen Alkoholen oder auch von mehrbasischen Carbonsäuren hergestellt worden sind. Als mehrwertige Alkohole kommen insbesondere 2- bis 4-funktionelle Alkohole und insbesondere Diole wie Ethlyenglykol und/oder 1,2-Propandiol, besonders aber das Glycerin als dreiwertiger Alkohol in Betracht. Geeignete mehrbasische Carbonsäuren sind Dicarbonsäuren mit 2 bis 10, vorzugsweise mit 2 bis 6 C-Atomen.

Werden Oligoester der geschilderten Art durch Kokondensation von Hydroxycarbonsäuren und insbesondere mehrwertigen Alkoholen hergestellt, so entstehen letztlich Oligoester mit endständigen Hydroxylgruppen. Die Menge der mitverwendeten Alkohole bestimmt - in Abstimmung mit den Reaktionsbedingungen - das mittlere Molekulargewicht der anfallenden Oligoester. Entsprechend werden durch Kokondensation von Hydroxycarbonsäuren mit insbesondere Dicarbonsäuren bzw. reaktiven Dicarbonsäurederivaten Oligoester mit endständigen Carboxylgruppen gebildet. Im einzelnen gilt hier das bekannte Wissen zur Herstellung von Polyestern bzw. Copolyestern. Die Mitverwendung von Monoalkoholen und/oder Monocarbonsäuren führt zur Blockierung der endständigen Carbonsäurengruppierung bzw. der endständigen Hydroxylgruppe des jeweiligen Oligomeren.

Die erfindungsgemäß als Träger bevorzugten Oligoester werden durch Umsetzung eines oder mehrerer der genannten polyfunktionellen Alkohole bzw. der entsprechenden polyfunktionellen Carbonsäuren mit Hydroxycarbonsäuren der angegebenen Art im Molverhältnis von 1 : 1 bis 1 : 10 und vorzugsweise dabei im Bereich von 1 : 1 bis 1 : 3 hergestellt. Produkte dieser Art können sich dadurch auszeichnen, daß sie das eingangs geschilderte Aufgabenprofil der erfindungsgemäßen Lehre optimal erfüllen. So sind beispielsweise Milchsaure/Glycerin-Oligoester, die Milchsäure/Glycerin-Molverhältnisse im Bereich von 1 : 1 bis 5 : 1 und insbesondere im Bereich von 1 : 1 bis 3 : 1 als Reaktionsansatz aufweisen, besonders geeignete Trägermaterialien im Sinne der erfindungsgemäßen Lehre.

Die Bildung der Oligoester ist eine übliche Veresterungsreaktion, die in Gegenwart geeigneter Katalysatoren in an sich bekannter Weise durchgeführt wird. Geeignet sind insbesondere beim Einsatz der Trägermaterialien im Rahmen medizinischer Bindemittelsysteme als Veresterungskatalysatoren physiologisch unbedenkliche Säuren wie Phosphorsäure. Die niederen Hydroxycarbonsäuren können als Monomerverbindungen aber auch in Form ihrer Dimeren, beispielsweise als Glykolid bzw. als Lactid in die Oligoveresterung eingesetzt werden.

Als Trägermaterial bevorzugte Oligoester der genannten Art liegen als niedrig-viskose Umsetzungsprodukte mit Viskositäten im Bereich bis 40000 mPas bei Raumtemperatur und insbesondere mit Viskositäten bis höchstens 25000 mPas bei Raumtemperatur vor. Der Bereich von 10000 bis 20000 mPas (Raumtemperatur) kann als besonders geeigneter Viskositätsbereich angesehen werden.

Träger der erfindungsgemäß geschilderten Art werden mit den Organo-Borverbindungen als initiatorkomponente zweckmäßigerweise unter Ausschluß von Luftsauerstoff abgemischt. Als Organo-Borverbindungen kommen dabei insbesondere die Komponenten in Betracht, die in den eingangs erwähnten Schutzrechtsveröffentlichungen der Anmelderin im einzelnen aufgezählt sind. Gemeinsam ist diesen Initiatoren hohe Reaktivität bei Luftzutritt bei jedoch gleichzeitig deutlich verringerter Neigung zur Selbstentzündlichkeit. Ermöglicht wird damit eine leichte und ungefährliche Handhabung des Startersystems. Die erfindungsgemäß einzuset-

EP 0 502 921 B1

zenden Organo-Borverbindungen zeigen bei der Anwendung an der Luft nur einen langsamen Aktivitätsverlust. Sie können damit als getrennte Härterkomponente in Zweikomponenten-Klebstoff-Systemen vorteilhaft zum Einsatz kommen. Die borhaltigen Harter führen zu hohen Festigkeiten der Klebstoffbindungen bzw. der in situ gebildeten geformten Kunststoffteile. Gleichzeitig besteht die Möglichkeit, die Härtungsgeschwindigkeit zu beeinflussen und damit den in der Praxis gewünschten Verhältnissen anzupassen. Die Struktur der Initiator-Komponente gewährleistet schließlich gute Adhäsion des Klebstoffs bzw. Bindemittels an körpereigenem Material.

Als Borverbindungen kommen demnach zahlreiche bekannte bzw. auf bekannte Weise herstellbare Boralkyle in Frage. Typische Vertreter solcher Borverbindungen sind beispielsweise 9-Borabicyclo-[3.3.1]-nonan, Diisopinocampheylboran, Dicyclohexylboran, Thexylboran-(2,3-dimethyl-2-butylboran), 3,5-Dimethylborinan, Diisoamylboran. Unter diesen Verbindungen ist das zuerst genannte 9-Borabicyclo-[3.3.1]-nonan aus praktischen Gründen bevorzugt.

Eine Zusammenstellung der Herstellungsmöglichkeiten geeigneter Borverbindungen findet sich in der Monographie Herbert C. Brown, 1975 "Organic Synthesis via Boranes", Verlag John Wiley ε Sons. Als Initiatoren können weiterhin Hydroborierungsprdoukte von Dialkylboranen und Olefinen eingesetzt werden. Als Olefine sind denkbar Buten, Isobuten, Hexen, Cyclohexen, Vinylchlorid, Allylchlorid, Allylamin oder auch Methacrylsäuremethylester, Vinylacetat oder Crotonsäuremethylester. Unter den geeigneten Verbindungen sind beispielsweise erwähnenswert: Diisopinocampheylbutylbor, Thexylcyclohexylcyclopentylbor, Thexyllimonylbor, Trinorbornylbor, B-Butyl-9-borabicyclo[3.3.1]-nonan, B-Isobutyl-9-borabicyclo[3.3.1]-nonan, B-Isobutyl-9-borabicyclo[3.3.1]-nonan, B-2-(4-Cyclohexenyl)ethyl 9-borabicyclo[3.3.1]-nonan, B-Cyclopropyl-9-borabicyclo[3.3.1]-nonan, B-p-Tolyl-9-borabicyclo[3.3.1]-nonan, B-tert.-Butyl-3 ,5-dimethylborinan. Des weiteren sind Umsetzungsprodukte von 1,2-Dihydroxybenzolen wie Brenzcatechin mit Borwasserstoff (Catecholboran) und Tri-n-butylboroxin geeignet.

Zur Herstellung der Startersysteme werden die Organo-Borverbindungen zweckmäßigerweise unter völligem Sauerstoffausschluß in den Trägermaterialien auf Basis der Oligoester gelöst. Gegebenenfalls kann dabei unter mäßigem Erwärmen gearbeitet werden. So ist es beispielsweise möglich, zur Beschleunigung der Auflösung auf Temperaturen bis zu 100 °C, vorzugsweise bis zu etwa 70 °C zu erwärmen. Die Mitverwendung inerter flüssiger Lösungsmittel bei dem Vermischungsvorgang kann zweckmäßig sein. Geeignet sind hier die bekannten Lösungsmittel für Organo-Borverbindungen, insbesondere Tetrahydrofuran oder Polyether wie Diethylenglykoldimethylether, aber auch flüchtige Ester, Halogenkohlenwasserstoffe und dergleichen. Unter Mitverwendung dieser flüssigen Hilfsmittel werden innige Mischungen hergestellt, die Hilfsflüssigkeiten können dann abgezogen und dann die Startergemische isoliert werden. Ihre Lagerung erfolgt zweckmäßigerweise im verschlossenen Gefäß, bevorzugt unter Inertgas, beispielsweise unter Stickstoff.

Der Gehalt der Startersysteme an Organo-Borverbindungen wird zweckmäßigerweise so gewählt, daß der Borgehalt des Startersystems im Bereich von etwa 0,5 bis 10 Gew.-%, vorzugsweise im Bereich von etwa 1 bis 5 Gew.-% liegt. Diese auf das reine Bor bezogenen Maßzahlen regeln je nach Beschaffenheit und Molekülgröße der Organoborverbindung die Mischungsverhältnisse dieser im aktuellen Fall eingesetzten Organo-Borverbindungen und des erfindungsgemäß ausgewählten Trägermaterials. Für den praktischen Einsatz hat sich als Organo-Borverbindung insbesondere das 9-Borabicyclo[3.3.1]-nonan bewährt.

Die Startersysteme der Erfindung kommen in Systemen zum Einsatz, die durch radikalisch ausgelöste Polymerisation zur Umsetzung gebracht werden und im einzelnen in den eingangs zitierten druckschriftlichen Vorveröffentlichungen der Anmelderin ausführlich beschrieben sind. Nur kurz wird dementsprechend hier zusammengefaßt:

Geeignet können insbesondere Systeme auf Basis von Acrylatund/oder Methacrylatverbindungen sein - im folgenden als (Meth)acrylatverbindungen bezeichnet - die insbesondere aus den folgenden Komponenten bestehen:

einem oder mehreren radikalisch polymerisierbaren Monomeren, Polymeren zur Kohäsionsverbesserung und zur Einstellung der Viskosität,

gegebenenfalls aktiven Füllstoffen zur Verbesserung der mechanischen Eigenschaften

sowie dem Radikalstartersystem zur Polymerisationsauslösung.

Als polymerisierbare Monomere haben insbesondere (Meth)acrylatverbindungen in Kombination mit (Meth)acrylsäure überragende Bedeutung. Bekannt ist dabei, daß sich die Haftung von (Meth)acrylatklebstoffen auf Knochenmaterial durch Boralkylhärter steigern läßt. Vergleiche hierzu im einzelnen die in der DE 32 29 635 (A1) zitierte Literatur. In dieser zuletzt genannten Literaturstelle sind Bindemittelsysteme für chirurgische Zwecke beschrieben, die dadurch gekennzeichnet sind, daß sie als resorbierbare (Meth)acrylatkomponenten bei Raumtemperatur flüssige bis feste (Meth)acrylsäureester mit (Meth)acrylatresten an Polyester-Oligomerketten aus Hydroxycarbonsäuren zusammen mit Organo-Borverbindungen als Startern enthalten. Klebstoffsysteme der hier geschilderten Art sind für die erfindungs-

4

gemäß jetzt neu beschriebenen Startersysteme besonders geeignet. Dabei können entsprechende körperresorbierbare (Meth)acrylatkomponenten sowohl in der Monomerkomponente als auch in der üblicherweise mitverwendeten Polymerkomponente zum Einsatz kommen. Die Lehre der Erfindung ist allerdings nicht auf den Einsatz solcher körperresorbierbaren Materialien eingeschränkt. Typische Beispiele für solche Polymerkomponenten sind Polymethyl(meth)acrylat-Copolymere von Methyl(meth)acrylat Polychloropren, chlorsulfoniertes Polyethylen, Nitrilkautschuke und -urethane, die zur Verstärkung bzw. Elastifizierung und gleichzeitig als Verdickungsmittel Einsatz finden können. Werden körperabbaubare Systeme eingesetzt, sind sowohl in der ethylenisch ungesättigten Monomerkomponente wie in den gegebenenfalls zu verwendenden Polymerkomponenten Hydroxycarbonsäurereste. insbesondere entsprechende Reste der Glykolsäure und/oder der Milchsäure wesentliche Molekülbausteine.

In vielen Fällen kann es zweckmäßig oder notwendig sein, weitere Hilfsstoffe wie Füllstoffe, beispielsweise Quarzmehl oder dergleichen zuzugeben. Schließlich kann es zweckmäßig sein, mit geeigneten Farbstoffen bzw. Pigmenten einzufärben.

Als zusätzliche polymerisierbare Bestandteile können in den Kunststoffmassen die zahlreichen bekannten Verbindungen mit polymerisierbarer ethylenischer Doppelbindung eingesetzt werden, die üblicherweise in beispielsweise Gießharzen, Fullstoffen und insbesondere in Reaktionsklebstoffen zur Verwendung kommen. Geeignet sind beispielsweise (Meth)acrylsäureverbindungen mit monovalenten oder polyvalenten insbesondere 2wertigen Alkoholen, aber auch andere Derivate der (Meth)acrylsäure wie die entsprechenden Säureamide, die am Amidstickstoff beispielsweise mit Kohlenwasserstoffresten substituiert sein können.

Die Mischungsverhältnisse des erfindungsgemäßen Starter/Trägersystems zum Monomer-/Bindemittel-System liegen im üblichen Bereich. Beispielsweise können die erfindungsgemäßen Startersysteme in Mengen von etwa 0,5 bis 30 Gew.-% - bezogen auf den zu polymerisierenden Anteil - verwendet werden.

In den nachfolgenden Beispielen werden die Herstellung ausgesuchter Milchsäureoligomeren, ihre Abmischung mit 9-BBN(9-Borabicyclo[3.3.1]-nonan) sowie der Einsatz der so hergestellten Startersysteme beschrieben.

**Beispiele**

1. Herstellung des Oligomeren

Allgemeine Vorschrift zur Herstellung der Umsetzungsprodukte von Lactid mit Glycerin

Lactid (L(-)-Lactid N der Fa. Böhringer, Ingelheim) und Glycerin wurden in einer üblichen Laborapparatur unter Stickstoff und unter Rühren katalysiert mit 0,5 % Phosphorsäure, bezogen auf Lactid, innerhalb von einer Stunde auf 195 °C erwärmt. Man ließ dann 6 Stunden bei 195 °C reagieren und füllte heiß ab. Folgende Produkte wurden hergestellt:
a) Milchsäure : Glycerin 3 : 1

| Ansatz: | 432 g Lactid | = 3 Mol |
| | 184 g Glycerin | = 2 Mol |
| Ausbeute: | 610 g/99 % | |

b) Milchsäure : Glycerin 2 : 1

| Ansatz: | 1008 g Lactid | = 7 Mol |
| | 644 g Glycerin | = 7 Mol |
| Ausbeute: | 1621 g/98,2 % | |

Die Zusammensetzung der Produkte und ihre Eigenschaften sind in der Tabelle 1 angegeben.

2. Herstellung des Boralkylhärters

Allgemeine Herstellvorschrift zur Herstellung des Boralkylhärters aus Milchsäureoligomeren nach 1 und 9-BBN

In einem Dreihalskolben mit Rührer und Thermometer werden 100 g des Oligomeren nach 1a oder 1b vorgelegt. Nach Erwärmung auf ca. 50 °C wird mit einer Drehschieberpumpe 2 x 10 Minuten auf 1 mbar evakuiert und jeweils mit Stickstoff belüftet. Im Anschluß werden im Stickstoffstrom die in der Tabelle 2 angegebenen Mengen 9-Borabicyclo 3.3.1 -nonan (9 BBN) zugegeben. Zur besseren Reaktionsführung werden über einen Kolonnenkopf 100 ml THF (gelagert über $FeCl_2$) in das Reaktionsgemisch destilliert. Das Gemisch wird unter einem Stickstoffstrom 1,5 Stunden bei ca. 60 bis 65 °C gehalten. Danach wird das THF mittels Wasserstrahlvakuum bei ca. 60 °C abdestilliert und die Restmengen THF bei 1 mbar abgezogen. Das Reaktionsprodukt wird unter Stickstoffstrom in ein Vorratsgefäß überführt und unter $N_2$ verschlossen gelagert.

Die Herstellung der Härter und ihre Eigenschaften sind in der Tabelle 2 zusammengestellt.

3. Verwendung der dargestellten Boralkyle auf Oligomilchsäurebasis

Allgemeine Vorschrift

In einem Becherglas wurden 40 g Polymethacrylsäuremethylester (PMMA, handelsübliches Pulver, Plexigum MB 319 der Fa. RÖHM, Darmstadt) in 45 g Methacrylsäuremethylester (MMA) und 5 g Methacrylsäure (MAS) unter Rühren gelöst. Zu jeweils 10 g dieser Mischung wurden unter weiterem intensivem Rühren zwischen 1,5 und 10 Gew.-% der bereits unter 2) beschriebenen Boralkylstarter zugegeben. Die Topfzeiten der Mischungen variieren zwischen 4,5 und 9 Minuten. Mit diesen Klebstoffen wurden innerhalb der Topfzeit sandgestrahlte und entfettete Eisenbleche verklebt und nach 24 Stunden die Festigkeiten im Zugscherversuch nach DIN 53 281/3 gemessen. Die Ergebnisse sind in Tabelle 3 zusammengestellt.

Zum Nachweis der hohen Stabilität der dargestellten Boralkyle auf Oligomilchsäurebasis gegenüber Luftsauerstoff wurden sie in einer weiteren Versuchsreihe im offenen Gefäß zwischen 72 und 120 Stunden an der Luft gelagert und im Anschluß als Harter eingesetzt und getestet. Die Topfzeiten und Zugscherfestigkeiten sind in Tabelle 4 in Klammern gesetzt.

## Tabelle 1

Oligohydroxycarbonsäuren aus Glycerin und Lactid

| Bei-spiel | Glycerin | Lactid | Konsistenz bei Raumtemperatur | Viskosität Epprecht-Viskosimeter/MK 4 bei Raumtemperatur | % freie Milchsäure | % freies Glycerin | % freies Acrolein |
|---|---|---|---|---|---|---|---|
| a | 1 | 1,5 | klar, leicht viskos | 18000 mPas | | | |
| b | 1 | 1 | leicht gelb leicht, viskos | 16500 mPas | | | |

EP 0 502 921 B1

## Tabelle 2

### Darstellung der Initiatorkomponenten a 1, a 2, b

| Nr. | Oligomeren Milchsäure : Glycerin | eingesetzte Menge 9-BBN/g pro 100 g Substanz | Produkteigenschaften | Gew.-% Bor |
|---|---|---|---|---|
| a 1 | 3 : 1 | 41 | homogen, farblos viskos | 3,64 |
| a 2 | 3 : 1 | 20 | homogen, farblos viskos | 1,78 |
| b | 2 : 1 | 41 | homogen, hellgelb farblos | 3,64 |

## Tabelle 3

Übersicht über die gemessenen Zugfestigkeiten / $N/mm^2$

| Monomer-Klebstoffharz | Härterkomponente Bez. | Härterkomponente Zusatz in Gew.-% | Topfzeit in Min. | Festigkeit an Fe-Bleche nach 24 h RT/in $N/mm^2$ | B-Gehalt im Klebstoff/Gew.-% |
|---|---|---|---|---|---|
| 10 g | a 1 | 1,5 | 9 | 29 | 0,055 |
| 10 g | | 3 | 5 (7) | 27 (27) | 0,11 |
| 10 g | | 5 | 5 | 32 | 0,18 |
| 10 g | | 10 | 4,5 | 26 | 0,36 |
| 10 g | a 2 | 1,5 | 9 | 0 | 0,055 |
| 10 g | | 3 | 6 (7) | 27 (26) | 0,11 |
| 10 g | | 5 | 5 | 27 | 0,18 |
| 10 g | | 10 | 4,5 | 25 | 0,36 |
| 10 g | b | 1,5 | 9 | 20 | 0,055 |
| 10 g | | 3 | 7,5 | 23 | 0,11 |
| 10 g | | 5 | 7 | 25 | 0,18 |
| 10 g | | 10 | 7 | 22 | 0,36 |

EP 0 502 921 B1

**Patentansprüche**

1. Startersystem für die Polymerisationsauslösung ethylenisch ungesättigter Verbindungen auf Basis von Sauerstoff-reaktiven Organo-Borverbindungen in Kombination mit Sauerstoff-resistenten Trägern, dadurch gekennzeichnet, daß sie als Träger Oligoester von niederen Hydroxycarbonsäuren mit 2 bis 10 C-Atomen enthalten.

2. Startersystem nach Anspruch 1, dadurch gekennzeichnet, daß als Träger Oligoester von Hydroxycarbonsäuren mit 2 bis 6 C-Atomen und insbesondere Oligoester der Glykolsäure und/oder Milchsäure vorliegen.

3. Startersystem nach Anspruchen 1 und 2, dadurch gekennzeichnet, daß als Träger mit Hydroxyl- und/oder Carboxylgruppen terminierte Oligoester der niederen Hydroxycarbonsäuren vorliegen.

4. Startersystem nach Anspruchen 1 bis 3, dadurch gekennzeichnet, daß die Träger unter Mitverwendung von Alkoholen und/oder Carbonsäuren, vorzugsweise unter Einsatz mehrfunktioneller Alkohole oder ein- und/oder mehrfunktioneller Carbonsäuren hergestellt worden sind.

5. Startersystem nach Anspruchen 1 bis 4, dadurch gekennzeichnet, daß die Träger Oligoester aus der Umsetzung niederer 2- und/oder 3-funktioneller Alkohole, insbesondere entsprechende Umsetzungsprodukte des Ethylenglykols und/oder des Glycerins mit den Hydroxycarbonsäuren bzw. ihren reaktionsfähigen Derivaten sind.

6. Startersystem nach Ansprüchen 1 bis 5, dadurch gekennzeichnet, daß Oligoester als Träger vorliegen, die durch Umsetzung von polyfunktionellen Alkoholen bzw. Carbonsäuren mit den Hydroxycarbonsäuren oder ihren reaktionsfähigen Derivaten im Molverhältnis von 1 : 1 bis 1 : 10, vorzugsweise in Molverhältnissen von 1 : 1 bis 1 : 3 hergestellt worden sind.

7. Startersystem nach Ansprüchen 1 bis 6, dadurch gekennzeichnet, daß sie als Oligoester niedrig-viskose Umsetzungsprodukte, bevorzugt mit Viskositäten im Bereich bis 40000 mPas, insbesondere bis maximal 25000 mPas - bestimmt jeweils bei Raumtemperatur - enthalten, wobei entsprechend niedrig-viskose Umsetzungsprodukte von Glycerin mit Glykolsäure und/oder insbesondere Milchsäure besonders bevorzugt sein können.

8. Startersystem nach Ansprüchen 1 bis 7, dadurch gekennzeichnet, daß Mischungsverhältnisse von Träger zu Organo-Borverbindungen vorliegen, daß der Bor-Gehalt des Startersystems im Bereich von 0,5 bis 10 Gew.-%, vorzugsweise im Bereich von 1 bis 5 Gew.-% liegt.

9. Startersystem nach Ansprüchen 1 bis 8, dadurch gekennzeichnet, daß sie Organo-Borverbindungen enthalten, die an Luft praktisch keine Selbstentzündlichkeit besitzen, gleichzeitig bei Raumtemperatur oder nur mäßig erhöhten Temperaturen unter Luftzutritt zur Polymerisationsauslösung befähigt sind.

10. Startersystem nach Ansprüchen 1 bis 9, dadurch gekennzeichnet, daß sie Alkyl- und/oder Arylreste aufweisende Organo-Borverbindungen enthalten, die auch B-H-Bindungen besitzen können.

11. Startersystem nach Ansprüchen 1 bis 10, dadurch gekennzeichnet, daß sie wenigstens eine Bortrialkylverbindung und/oder wenigstens eine Boralkylhydridverbindung - in diesem Fall insbesondere ein Dialkylborhydrid - enthalten.

12. Verwendung der Startersysteme nach Ansprüchen 1 bis 11 in Klebstoffsystemen des chirurgischen und/oder zahnmedizinischen Bereichs zur Aushärtung von körperresorbierbaren oder körperresistenten Klebern und/oder Zementen bzw. zur Ausbildung von Kunststoff-Formteilen.

**Claims**

1. An initiator system for initiating the polymerization of ethylenically unsaturated compounds, said system being based on oxygen-reactive organoboron compounds in combination with oxygen-resistant carriers, characterized in that it contains, as carriers, oligoesters of lower hydroxycarboxylic acids having from 2 to 10 carbon atoms.

2. The initiator system according to claim 1, characterized in that the carriers are oligoesters of hydroxycarboxylic acids having from 2 to 6 carbon atoms, and especially oligoesters of glycolic acid and/or lactic acid.

3. The initiator system according to claims 1 and 2, characterized in that the carriers are hydroxyl and/or carboxyl-group terminated oligoesters of the lower hydroxycarboxylic acids.

4. The initiator system according to claims 1 to 3, characterized in that the carriers have been prepared with the concomitant use of alcohols and/or carboxylic acids, preferably using polyfunctional alcohols or mono and/or polyfunctional carboxylic acids.

5. The initiator system according to claims 1 to 4, characterized in that the carriers are oligoesters from the reaction of lower di- and/or trifunctional alcohols, especially the respective reaction products of ethylene glycol and/or glycerol with the hydroxycarboxylic acids and/or their reactive derivatives.

6. The initiator system according to claims 1 to 5, characterized in that the carriers are oligoesters from the reaction of polyfunctional alcohols and/or carboxylic acids, respectively, with hydroxycarboxylic acids or their reactive derivatives in a molar ratio of from 1:1 to 1:10, and preferably in molar ratios of from 1:1 to 1:3.

7. The initiator system according to claims 1 to 6, characterized in that it contains, as oligoesters, low-viscosity reaction products, preferably having viscosities within a range of up to 40,000 mPa·s, and especially of up to at most 25,000 mPa·s - each determined at room temperature - ,whereby the respective low-viscosity reaction products of glycerol with glycolic acid and/or especially lactic acid may be preferred.

8. The initiator system according to claims 1 to 7, characterized in that the mixing ratios of carrier to the organoboron compound are such that the boron content of the initiator system is within the range of from 0.5 to 10% by weight, and preferably within the range of from 1 to 5% by weight.

9. The initiator system according to claims 1 to 8, characterized in that it contains organoboron compounds which are not self-igniting in air and at same time are capable of initiating the polymerization in the presence of air at room temperature or only moderately elevated temperature.

10. The initiator system according to claims 1 to 9, characterized in that it contains organoboron compounds which comprise alkyl and/or aryl groups and may also contain B-H bonds.

11. The initiator system according to claims 1 to 10, characterized in that it contains at least one trialkyl-boron compound and/or at least one alkylboron hydride compound - in this case especially a dialkyl boron hydride.

12. Use of the initiator system according to claims 1 to 11 in adhesive systems in the areas of surgery and/or dentistry for curing body-resorbable or body-resistant adhesive agents and/or cements or for forming molded parts of synthetic materials.


**Revendications**

1. Système d'induction pour le démarrage de la polymérisation de composés éthyléniquement insaturés à base de composés organo-borés réactifs à l'oxygène en combinaison avec des supports résistant à l'oxygène, caractérisé en ce qu'ils renferment comme support des oligoesters d'acides hydroxycarboxyliques inférieurs ayant de 2 à 10 atomes de carbone.

2. Système d'induction selon la revendication 1, caractérisé en ce que comme support des oligoesters d'acides hydroxycarboxyliques ayant de 2 à 6 atomes de carbone et en particulier des oligoesters d'acide glycolique et/ou d'acide lactique, sont présents.

3. Système d'induction selon les revendications 1 et 2, caractérisé en ce que comme support, des oligoesters terminés par des groupes hydroxyle et/ou carboxyle, des acides hydroxycarboxyliques inférieurs sont présents.

4. Système d'induction selon les revendications 1 à 3, caractérisé en ce que les supports sont produits en utilisant conjointement des alcools et/ou des acides carboxyliques, de préférence en utilisant des alcools plurifonctionnels ou des acides carboxyliques mono et/ou plurifonctionnels.

5. Système d'induction selon les revendications 1 à 4, caractérisé en ce que les supports sont des oligoesters provenant de la réaction d'alcools inférieurs di- et/ou trifonctionnels, en particulier des produits de réaction correspondants de l'éthylèneglycol et/ou du glycérol avec des acides hydroxycarboxyliques ou leurs dérivés aptes à la réaction.

6. Système d'induction selon les revendications 1 à 5, caractérisé en ce que des oligoesters sont présents comme support, oligoesters qui ont été produits par réaction d'alcools ou d'acides carboxyliques polyfonctionnels avec les acides hydroxycarboxyliques ou leurs dérivés aptes à la réaction dans un rapport molaire de 1 : 1 à 1 : 10, de préférence dans des rapports molaires de 1 : 1 à 1 : 3.

7. Système d'induction selon les revendications 1 à 6, caractérisé en ce qu'ils renferment comme oligoesters des produits de réaction faiblement visqueux, de préférence ayant des viscosités dans la gamme allant jusqu'à 40000 mPas, en particulier jusqu'à, au maximum 25000 mPas déterminées respectivement à température ambiante, parmi lesquels les produits de réaction faiblement visqueux d'une manière correspondante, du glycérol avec l'acide glycolique et/ou en particulier l'acide lactique peuvent être particulièrement préférés.

8. Système d'induction selon les revendications 1 à 7, caractérisé en ce que les rapports de mélange du support aux composés organo-borés, sont présents de sorte que la teneur en bore du système d'induction se situe dans la gamme de 0,5 à 10 % en poids, de préférence dans la gamme de 1 à 5 % en poids.

9. Système d'induction selon les revendications 1 à 8, caractérisé en ce qu'ils renferment des composés organo-borés qui ne possèdent pratiquement aucune auto-inflammabilité à l'air et simultanément à température ambiante ou à des températures seulement modérément accrues tout en introduisant de l'air, sont aptes au démarrage de la polymérisation.

10. Système d'induction selon les revendications 1 à 9, caractérisé en ce qu'ils renferment des composés organiques du bore qui possèdent des radicaux alcoyle et/ou aryle, qui peuvent posséder aussi des liaisons B-H.

11. Système d'induction selon les revendications 1 à 10, caractérisé en ce qu'ils renferment au moins un composé de trialcoylbore et/ou au moins un composé boroalcoylhydrure, dans ce cas en particulier un dialcoylborohydrure.

12. Utilisation des Systèmes d'induction selon les revendications 1 à 11 dans des systèmes de colle du domaine chirurgical et/ou de la médecine dentaire pour le durcissement complet de colles et/ou de ciments résorbables dans le corps ou résistantes dans le corps, ou pour la formation de pièces de moulage en matière plastique.